# EUROPEAN PATENT SPECIFICATION

(11) **EP 2 731 594 B1**
(45) Date of publication and mention of the grant of the patent: **24.04.2019**
(21) Application number: 12814306.2
(22) Date of filing: 11.07.2012
(51) Int. Cl.: A61K 9/48, A61K 31/47, A61K 31/4704, C07D 215/18

(54) **CAPSULE FORMULATION COMPRISING MONTELUKAST AND LEVOCETIRIZINE**
KAPSELFORMULIERUNG MIT MONTELUKAST UND LEVOCETIRIZIN
PRÉPARATION SOUS FORME DE CAPSULE COMPRENANT DU MONTÉLUKAST ET DE LA LÉVOCÉTIRIZINE

(30) Priority: 15.07.2011 KR 20110070680; 28.10.2011 KR 20110111132
(43) Date of publication of application: 21.05.2014
(73) Proprietor: Hanmi Pharm. Co., Ltd., Hwaseong-si, Gyeonggi-do 445-910 (KR)
(72) Inventor: KIM, Yong Il, Suwon-si Gyeonggi-do 442-718 (KR); KIM, Dong Ho, Seoul 151-926 (KR); KWON, Taek Kwan, Suwon-si Gyeonggi-do 440-825 (KR); KIM, Kyeong Soo, Suwon-si Gyeonggi-do 440-710 (KR); PARK, Jae Hyun, Suwon-si Gyeonggi-do 443-470 (KR); WOO, Jong Soo, Suwon-si Gyeonggi-do 440-330 (KR)
(74) Representative: Marks & Clerk (Luxembourg) LLP
(86) International application number: PCT/KR2012/005506
(87) International publication number: WO 2013/012199

(56) References cited:
- WO-A1-2010/107404
- KR-A- 20090 017 423
- US-A- 5 698 558
- US-A1- 2006 034 928
- "A PHARMACEUTICAL COMPOSITION COMPRISING MONTELUKAST SODIUM AND LEVOCETIRIZINE DIHYDROCHLORIDE IN A SINGLE DOSAGE FORM", IP.COM JOURNAL, IP.COM INC., WEST HENRIETTA, NY, US, 15 May 2008 (2008-05-15), XP013124986, ISSN: 1533-0001
- R.T. RATHOD ET AL.: 'FDC of montelukast with levocetirizine: focus on bilayer technology' JOURNAL OF INDIAN MEDICAL ASSOCIATION vol. 107, no. 8, August 2009, pages 562 - 564, XP008173253

## Description

### FIELD OF THE INVENTION

The present invention relates to a capsule formulation for preventing or treating allergic rhinitis and asthma, which comprises two separate layers of: (1) a Montelukast layer comprising montelukast or a pharmaceutically acceptable salt thereof; and (2) a Levocetirizine layer comprising levocetirizine or a pharmaceutically acceptable salt thereof; and a method for the preparation thereof.

### BACKGROUND OF THE INVENTION

"Allergic rhinitis" refers to a symptomatic disorder of the nose induced by an IgE-mediated inflammation after allergen exposure of the membrane of the nose. The allergic rhinitis includes such symptoms as rhinorrhea, nasal obstruction, nasal itching, sneezing, ocular pruritis and so on.

"Asthma" refers to a disorder wherein inflammation of the airways causes bronchial mucosa to swell and muscular convulsion to occur in bronchi which restricts airflow into and out of the lungs, and hence. Asthma may cause such symptoms as shortness of breath, severe coughing, and in severe cases, status asthmaticus, which may result in even death.

Allergic rhinitis and asthma may develop separately; however, there is a study showing that approximately 60% of patients with allergic rhinitis have asthma as well and that 85∼95% of patients with asthma also suffer from allergic rhinitis, indicating high rates of complications between said two patient groups. Thus, there has been a need for developing a complex composition, which has an improved stability and efficacy for treatment of said two conditions.

Meanwhile, Montelukast is an antagonist inhibits cysteinyl leukotriene receptor (CysLT1), which is used for prevention and treatment of leukotriene-mediated diseases. Particularly, it has been reported that montelukast is effective in the treatment of allergic rhinitis, atopic dermatitis, chronic urticaria, sinusitis, nasal polyp, chronic obstructive pulmonary disease, conjunctivitis including nasal conjunctivitis, migraine, cystic fibrosis, viral bronchiolitis, and the like [*see, e.g.,* S. E. Dahlen, Eur. J. Pharmacol., 533(1-3), 40-56(2006)]. Further, Singulair (MSD) comprising montelukast sodium is approved for treating asthma in adults and pediatric patients of 2 years plus, and currently available in the market.

Cetirizine is (2-(4-((4-chlorophenyl)phenylmethyl)-1-piperazinyl)ethoxy-acetic acid, and its levorotatory and dextrorotatory mirror image enantiomers were disclosed as "Levocetirizine" and "Dextrocetirizine ", respectively.

Levocetirizine can be obtained by degradation or asymmetric synthesis from a racemic mixture of Cetirizine, e.g., conventional methods disclosed in UK Patent No. 225321, or enzymatic biocatalytic hydrolysis disclosed in US Patent Nos. 4800162 and 5057427. Levocetirizine possesses antihistamine properties and hence is useful as an antiallergenic, an antihistamine agent, as well as an anticonvulsant and a bronchodialator.

International Publication No. WO 94/06429 discloses a method of treating seasonal and perennial allergic rhinitis using Levocetirizine. Also, Korean Patent No. 926410 discloses a pharmaceutical composition for treatment of allergic diseases, which comprises a segment comprising Cetirizine and a segment comprising pseudoephedrine, as active ingredients. However, the efficacy of said active ingredients, Cetirizine and pseudoephedrine, against allergic disease asthma has not been proven. Besides, consistent use of nasal decongestant, pseudoephedrine may exacerbate nasal congestion due to the rebound reaction, and may cause intractable drug induced rhinitis. Therefore, it is not recommended for more than 2 weeks of usage thereof.

Further, there has been a report relating to a pharmaceutical composition in a bilayer tablet form comprising montelukast sodium, which is stable in a basic condition, and levocetirizine dihydrochloride, which is stable in an acidic condition [R. T. Rathod, J. Indian Med. Assoc., 107(8), 562-564 (2009)]. In the preparation of said composition in a tablet form, it is very difficult to completely separate montelukast and levocetirizine from each other. Even in case a bilayer tablet is formed, it is impossible to mechanically separate each active ingredient completely. Moreover, a bilayer tablet machine is required in order to manufacture such tablets. The technical disclosure IPCOM000170324D in the PriorArtDatabase ip.com relates to pharmaceutical compositions comprising montelukast sodium and levocetirizine dihydrochloride in a single dosage form, designed to minimize interaction between the two drugs.

In addition, Montelukast is known to be unstable when exposed to light, heat, or moisture, and yields such degraded products as montelukast sulfoxide of Formula (I) and montelukast *cis*-isomer of Formula (II). According to M. M. Al Omari *et al*., when a commercially available Singulair chewable tablet was exposed to sunlight, the amount of montelukast sulfoxide was increased by 2.4% after 3 weeks; and when motelukast in 0.1 M hydrochloric acid solution was exposed to sodium, the amount of montelukast *cis*-isomer was increased by *14.6% [see* M. M. Al Omari et at., J. Pharm. And Biomed., 45, 465-471 (2007)]. As shown in the report, it is not easy to prepare a stable montelukast product against aging.

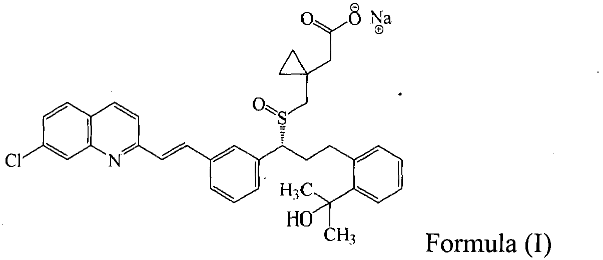

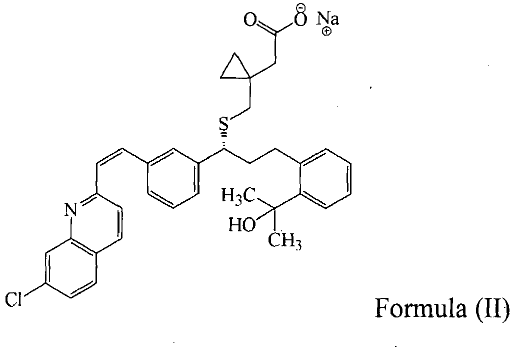

Levocetirizine is also instable in terms of physiochemical properties, and it is difficult to prepare a stable product against aging. There are three major degradation products of Levocetirizine, which include related compound A of Formula (III), related compound B of Formula (IV), and related compound D of Formula (V). Related compounds A and B are created via hydrolysis of Levocetirizine, and related compound D is created via ethyl ester addition of Levocetirizine. In fact, Levocetirizine shows an increased rate of formation of related compounds A, B, and D under accelerated stability conditions, and hence it is not easy to provide prescription stability.

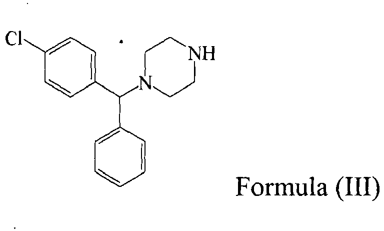

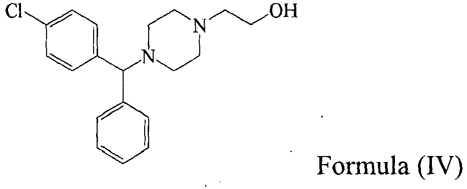

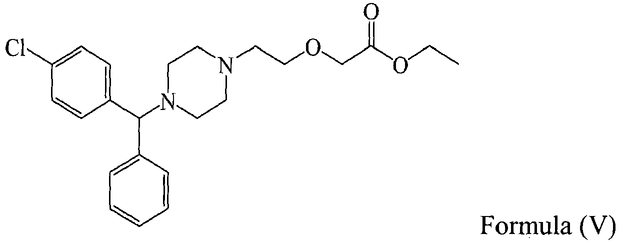

Therefore, the present inventors have developed a pharmaceutical composition comprising Montelukast as an anti-leukotriene agent with good stability while exerting no adverse effects; and Levocetirizine as an anti-histamine agent which is effective on early allergic reaction, for treatment of allergic disorders including allergic rhinitis and asthma, having stability for long-term use.

### SUMMARY OF THE INVENTION

Therefore, it is an object of the present invention to provide a pharmaceutical formulation according to claim 1 for preventing or treating allergic rhinitis and asthma, which comprises montelukast or a pharmaceutically acceptable salt thereof; and levocetirizine or a pharmaceutically acceptable salt thereof.

It is another object of the present invention to provide a method according to claim 16 for preparing the pharmaceutical formulation.

In accordance with the object of the present invention, there is provided a capsule formulation for preventing or treating allergic rhinitis and asthma, which comprises two separate layers of:
(1) a Montelukast layer comprising montelukast or a pharmaceutically acceptable salt thereof; and
(2) a Levocetirizine layer comprising levocetirizine or a pharmaceutically acceptable salt thereof, wherein said Montelukast layer and said Levocetirizine layer contain water in an amount of 5% or less.

In accordance with another object of the present invention, there is provided a method for preparing the capsule formulation, which comprises the steps of:
(i) mixing montelukast or a pharmaceutically acceptable salt thereof and a pharmaceutically acceptable additive, and granulating the mixture to obtain granules or forming the granules into a tablet;
(ii) mixing levocetirizine or a pharmaceutically acceptable salt thereof and a pharmaceutically acceptable additive, and granulating the mixture to obtain granules or forming the granules into a tablet; and
(iii) filling said tablet or granules of montelukast prepared in step (i) and said tablet or granules of levocetirizine prepared in step (ii) into a hard capsule to form separate layers in the capsule, wherein said Montelukast layer and said Levocetirizine layer contain water in an amount of 5% or less.

### BRIEF DESCRIPTION OF THE DRAWING

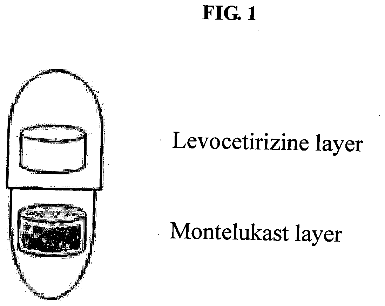

The above and other objects and features of the present invention will become apparent from the following description of the invention, when taken in conjunction with accompanying Figure 1 which shows a schematic view of the capsule formulation of the present invention.

### DETAILED DESCRIPTION OF THE INVENTION

Hereinafter, the present invention is explained in detail.

The present invention provides a capsule formulation for preventing or treating allergic rhinitis and asthma, which comprises two separate layers of: (1) a Montelukast layer comprising montelukast or a pharmaceutically acceptable salt thereof; and (2) a Levocetirizine layer comprising levocetirizine or a pharmaceutically acceptable salt thereof, wherein said Montelukast layer and said Levocetirizine layer contain water in an amount of 5% or less.

In the present invention, the Montelukast layer and Levocetirizine layer may independently be in the form of granules or a tablet. Specifically, the capsule formulation of the present invention is a capsule formulation prepared by filling (1) a tablet or granules of montelukast comprising montelukast or a pharmaceutically acceptable salt thereof; and (2) a tablet or granules of levocetirizine comprising levocetirizine or a pharmaceutically acceptable salt thereof into a hard capsule to form two separate layers in the capsule. Wherein, at least one of the Montelukast layer and Levocetirizine layer may be in the form of a tablet.

In order to prevent any undesired side reactions caused by water, said Montelukast layer and Levocetirizine layer may be prepared without employing water or an organic solvent, or prepared in a condition which contains substantially no water or organic solvent. The amount of water content in the Montelukast layer and Levocetirizine layer is 5% or less, respectively.

The capsule formulation of the present invention employs an antihistamine agent levocetirizine as a first active ingredient to reduce early allergic reaction, as well as an anti-leukotriene agent montelukast as a second active ingredient to treat and prevent one of the major symptoms of late allergic rhinitis, *i.e.* nasal obstruction and asthma.

Montelukast or the pharmaceutically acceptable salt thereof used as a first active ingredient in the present invention is preferably montelukast sodium. The daily dosage amount of montelukast or the pharmaceutically acceptable salt thereof is 0.4 to 100 mg, preferably 1 to 50 mg, more preferably 2.5 to 20 mg per unit dosage form.

Levocetirizine or the pharmaceutically acceptable salt thereof used as a second active ingredient in the present invention is, for example, disclosed in European Patent Applicaion. Nos. 0058146, 0601028 and 0801064, UK Patent Nos. 2225320 and 2225321, US Patent No. 5478941, and International Patent Publication No. WO 97/37982. The pharmaceutically acceptable salt of levocetirizine may include, but not limited to, an acid-addition salt of the pharmaceutically acceptable non-toxic organic or inorganic acid, such as salts of acetic acid, citric acid, maleic acid, succinic acid, ascorbic acid, hydrochloric acid, hydrobromic acid, sulfuric acid, phosphoric acid and the like; a metal salt (*e.g.,* sodium salt or calcium salt), ammonium salt, amine salt, and amino acid salt, preferably levocetirizine dihydrochloride salt. The daily dosage amount of levocetirizine or the pharmaceutically acceptable salt thereof is 0.4 to 100 mg, preferably 1 to 50 mg, more preferably 2.5 to 20 mg per unit dosage form.

Two active ingredients according to the present invention have rapid onset time, suitable dosage amount and less harmful side effects, and thus they can be applicable to pediatric patients and show good tolerability and safety even long term use.

In the capsule formulation of the present invention, the Montelukast layer and the Levocetirizine layer, specifically, the tablet or granules forming each of said layers may comprise a pharmaceutically acceptable diluent. Suitable examples of the diluent may include microcrystalline cellulose, lactose, ludipress, mannitol, calcium phosphate monobasic, starch, low-substituted hydroxypropyl cellulose, and a mixture thereof. The diluent may be used in an amount ranging from about 1 to about 99 % by weight, preferably about 5 to about 95 % by weight based on the total weight of the tablet or granules.

Additionally, the tablet or granules forming each of said layers further comprise a pharmaceutically acceptable additive, *e.g.,* a disintegrant, a binder, a stabilizing agent, a lubricant, a colorant, and the like.

Examples of the disintegrant may include any material showing a stable disintegration in a liquid environment, which is selected from the group consisting of crospovidone, sodium starch glycolate, croscarmellose sodium, low-substituted hydroxypropyl cellulose, startch, alginate or a sodium salt thereof, and a mixture thereof. Preferably, the disintegrant may be crospovidone, sodium starch glycolate, croscarmellose sodium, low-substituted hydroxypropyl cellulose or a mixture thereof. The disintegrant may be used in an amount ranging from 1 to 30 % by weight, preferably 2 to 15 % by weight based on the total weight of the tablet or granules.

Examples of the binder may include hydroxypropyl cellulose, hypromellose (hydroxypropyl methylcellulose), polyvinyl pyrrolidone, copovidone, macrogol, light anhydrous silicic acid, synthetic aluminum silicate, silicate derivatives such as calcium silicate or magnesium metasilicate aluminate, phosphate salts such as calcium phosphate dibasic, carbonate salts such as calcium carbonate, and a mixture thereof. The binder may be used in an amount ranging from 1 to 30 % by weight, preferably 2 to 20 % by weight based on the total weight of the tablet or granules.

The stabilizing agent used in the present invention may be preferably an antioxidant. The employment of the antioxidant reduces undesired side reactions caused by temperature and moisture, and thus, enhances stability against aging effects. Specific examples of the antioxidant may include butylated hydroxytoluene (BHT), butylated hydroxyanisole (BHA), ascorbic acid, ascorbyl palmitate, ethylenediaminetetraacetic acid (EDTA), sodium pyrosulfite, and a mixture thereof, preferably butylated hydroxytoluene. The stabilizing agent may be used in an amount ranging from 0.01 to 10% by weight, preferably 0.1 to 5% by weight based on the total weight of the tablet or granules.

Examples of the lubricant may include stearic acid, metal salts of stearic acid such as calcium stearate or magnesium stearate, talc, colloid silica, sugar fatty acid ester, hydrogenated vegetable oil, high melting point wax, glyceryl fatty acid ester, glycerol dibehenate and a mixture thereof. The lubricant may be used in an amount ranging from 0.3 to 5 % by weight, preferably 0.5 to 3% by weight based on the total weight of the tablet or granules.

Further, each tablet comprising montelukast or levocetirizine layer may further comprise a coating layer. The coating layer may be formed on the surface of at least one selected from said tablets so as to completely separate montelukast and levocetirizine. At this time, in order to improve stabilities of monelukast and levocetirizine, the coating layer may be prepared without employing water or an organic solvent, or prepared as a water-based coating which substantially contains no water or organic solvent

In the present invention, the coating substrate used for the coating layer may be conventional high molecular compounds. Examples of the coating substrate may include methylcellulose, ethylcellulose, polyvinyl alcohol, polyvinylpyrrolidone, hydroxyethylcellulose, hypromellose, but not limited thereto. The amount of the coating substrate is preferably kept at minimum so as to improve efficiency in production and provide the formulation of a size optimal for administration. Therefore, the coating substrate may be used in an amount ranging from 1 to 20 % by weight, preferably 2 to 10% by weight based on the total weight of the tablet or granules.

In the capsule formulation of the present invention, the capsule may be any conventional hard capsules that are generally used in the preparation of medicine. The hard capsule substrates used in the present invention may include, e.g., gelatin, hypromellose, pullulan (NP caps™, etc; Capsugel), or polyvinyl alcohol. In the present invention, hypromellose or pullulan having low water content is preferable to minimize degradation of active ingredients caused by water.

In the present invention, the hard capsules may have any conventional capsule size used in the preparation of medicine. The internal volume varies with size of hard capsules: No. 00 (0.95 mL), No. 0 (0.68 mL), No. 1 (0.47 mL), No. 2 (0.37 mL), No. 3 (0.27 mL) and No. 4 (0.20 mL). The size of the capsule is preferably small for patients' convenience, however, due to mass limit of the contents to be filled in the capsule, the size of the capsule used in the present invention may include No. 0, No. 1, No. 2, No. 3, and No. 4, preferably No. 1, No. 2, and No. 3.

In one embodiment of the present invention, the capsule formulation comprises (a) a montelukast tablet comprising montelukast or a pharmaceutically acceptable salt thereof; and (b) a levocetirizine tablet comprising levocetirizine or a pharmaceutically acceptable salt thereof, wherein the tablets are filled into the hard capsule.

In another embodiment of the present invention, the capsule formulation comprises (a) montelukast granules comprising montelukast or a pharmaceutically acceptable salt thereof; and (b) a levocetirizine tablet comprising levocetirizine or a pharmaceutically acceptable salt thereof, wherein the granules and the tablet are filled into the hard capsule.

In a further embodiment of the present invention, an inventive capsule formulation comprises (a) a montelukast tablet comprising montelukast or a pharmaceutically acceptable salt thereof; and (b) levocetirizine granules comprising levocetirizine or a pharmaceutically acceptable salt thereof, wherein the tablet and the granules are filled into the hard capsule.

The capsule formulation of the present invention may be used for preventing or treating allergic rhinitis and asthma, and the allergic rhinitis may include symptoms such as rhinorrhea, nasal obstruction, nasal itching, sneezing, ocular pruritis, and the like.

Further, the present invention provides a method for preparing the capsule formulation, which comprises the steps of: (i) mixing montelukast or a pharmaceutically acceptable salt thereof and a pharmaceutically acceptable additive, and granulating the mixture to obtain granules or forming the granules into a tablet; (ii) mixing levocetirizine or a pharmaceutically acceptable salt thereof and a pharmaceutically acceptable additive, and granulating the mixture to obtain granules or forming the granules into a tablet; and (iii) filling said tablet or granules of montelukast prepared in step (i) and said tablet or granules of levocetirizine prepared in step (ii) into a hard capsule to form separate layers in the capsule.

In steps (i) and (ii), the tableting process of the granules may be performed according to the conventional tableting methods with using a tablet machine. The tablet prepared may have a suitable hardness, e.g., in the range of 1 to 30 kp of the average hardness. The average hardness may be measured before forming any film coating layer on the tablets. Also, in case a tablet is produced in step (i) or (ii), the step may further comprise a process of coating the tablet.

In step (iii), the tablet or granules of montelukast, and the tablet or granules of levocetirizine may be filled into the hard capsule to form separate layers, wherein at least one selected from the Montelukast layer and the Levocetirizine layers may be in the form of a tablet.

The capsule formulation prepared in the present invention may be administered by oral, lingual, or sublingual routes.

The capsule formulation of the present invention comprises montelukast and levocetirizine separately in the hard capsule, and thus completely separate said two active ingredients. Therefore, the reactivity between two active ingredients can be minimized and the stability of the formulation is enhanced, thus optimizing therapeutic efficacy. It is also advantageous because preexisting analytical method for the evaluation of time-dependent stability of a single formulation can be also used for the inventive formulation, instead of developing a new analytical method.

The following Examples are intended to further illustrate the present invention without limiting its scope.

### Example 1: Preparation of Capsule formulation I

| Montelukast Layer | Quantity |
|---|---|
| Montelukast Sodium | 10.4 mg (Montelukast, 10 mg) |
| D-Mannitol | 74.3 mg |
| Microcrystalline Cellulose | 74.3 mg |
| Light anhydrous silicic acid | 5.0 mg |
| Hydroxypropyl Cellulose | 4.0 mg |
| Sodium Starch Glycolate | 30.0 mg |
| Magnesium Stearate | 2.0 mg |

| Levocetirizine Laver | Quantity |
|---|---|
| Levocetirizine Dihydrochloride | 5.0 mg |
| Ludipress | 60.5 mg |
| Microcrystalline Cellulose | 30.0 mg |
| Croscarmellose Sodium | 3.0 mg |
| Light anhydrous silicic acid | 0.5 mg |
| Magnesium Stearate | 1.0 mg |
| Opadry White (Y-1-7000) | 3.0 mg |
| Distilled Water | (15.0 mg) |

The ingredients described in Montelukast layer were mixed, and the mixture was pressed to a tablet using a round punch having a diameter of 5.5 mm to obtain a Montelukast tablet.

Meanwhile, the above procedure was repeated except for using the ingredients described in Levocetirizine layer to obtain a Levocetirizine tablet. Then, the Levocetirizine tablet was coated with a coating solution prepared by dissolving Opadry® White (Y-1-7000, Colorcon) in distilled water. Finally, said two tablets thus obtained were filled into a No. 1 hard capsule which is mainly composed of hypromellose, to obtain a capsule formulation comprising 10 mg of Montelukast and 5 mg of Levocetirizine.

### Example 2: Preparation of Capsule formulation II

| Montelukast Layer | Quantity |
|---|---|
| Montelukast Sodium | 5.2 mg (Montelukast, 5 mg) |
| D-Mannitol | 37.15 mg |
| Microcrystalline Cellulose | 37.15 mg |
| Light anhydrous silicic acid | 2.5 mg |
| Hydroxypropyl Cellulose | 2.0 mg |
| Sodium Starch Glycolate | 15.0 mg |
| Magnesium Stearate | 1.0 mg |

| Levocetirizine Layer | Quantity |
|---|---|
| Levocetirizine Dihydrochloride | 5.0 mg |
| Ludipress | 60.5 mg |
| Microcrystalline Cellulose | 30.0 mg |
| Croscarmellose Sodium | 3.0 mg |
| Light anhydrous silicic acid | 0.5 mg |
| Magnesium Stearate | 1.0 mg |
| Opadry White (Y-1-7000) | 3.0 mg |
| Distilled Water | (15.0 mg) |

The procedure of Example 1 was repeated except for using the ingredients and compositions described in Montelukast layer above, to obtain a capsule formulation comprising 5 mg of Montelukast and 5 mg of Levocetirizine.

### Example 3: Preparation of Capsule formulation III

| Montelukast Layer | Quantity |
|---|---|
| Montelukast Sodium | 5.2 mg (Montelukast, 5 mg) |
| D-Mannitol | 37.15 mg |
| Microcrystalline Cellulose | 37.15 mg |
| Light anhydrous silicic acid | 2.5 mg |
| Hydroxypropyl Cellulose | 2.0 mg |
| Sodium Starch Glycolate | 15.0 mg |
| Magnesium Stearate | 1.0 mg |

| Levocetirizine Layer | Quantity |
|---|---|
| Levocetirizine Dihydrochloride | 2.5 mg |
| Ludipress | 60.5 mg |
| Microcrystalline Cellulose | 30.0 mg |
| Croscarmellose Sodium | 3.0 mg |
| Light anhydrous silicic acid | 0.5 mg |
| Magnesium Stearate | 1.0 mg |
| Opadry White (Y-1-7000) | 3.0 mg |
| Distilled Water | (15.0 mg) |

The procedure of Example 1 was repeated except for using the ingredients and compositions described in Montelukast layer above and that the actual content of levocetirizine was 2.5 mg in Levocetirizine layer, to obtain a capsule formulation comprising 5 mg of Montelukast and 2.5 mg of Levocetirizine.

### Example 4: Preparation of Capsule formulation IV

| Montelukast Layer | Quantity |
|---|---|
| Montelukast Sodium | 4.16 mg (Montelukast, 4 mg) |
| D-Mannitol | 29.72 mg |
| Microcrystalline Cellulose | 29.72 mg |
| Light anhydrous silicic acid | 2.0 mg |
| Hydroxypropyl Cellulose | 1.6 mg |
| Sodium Starch Glycolate | 12.0 mg |
| Magnesium Stearate | 0.8 mg |

| Levocetirizine Layer | Quantity |
|---|---|
| Levocetirizine Dihydrochloride | 5.0 mg |
| Ludipress | 60.5 mg |
| Microcrystalline Cellulose | 30.0 mg |
| Croscarmellose Sodium | 3.0 mg |
| Light anhydrous silicic acid | 0.5 mg |
| Magnesium Stearate | 1.0 mg |
| Opadry White (Y-1-7000) | 3.0 mg |
| Distilled Water | (15.0 mg) |

The procedure of Example 1 was repeated except for using the ingredients and compositions described in Montelukast layer above, to obtain a capsule formulation comprising 4 mg of Montelukast and 5 mg of Levocetirizine.

### Example 5: Preparation of Capsule formulation V

| Montelukast Layer | Quantity |
|---|---|
| Montelukast Sodium | 10.4 mg (Montelukast, 10 mg) |
| D-Mannitol | 74.3 mg |
| Microcrystalline Cellulose | 74.3 mg |
| Light anhydrous silicic acid | 5.0 mg |
| Hydroxypropyl Cellulose | 4.0 mg |
| Sodium Starch Glycolate | 30.0 mg |
| Magnesium Stearate | 2.0 mg |
| Hypromellose | 1.73 mg |
| Hydroxypropyl Cellulose | 1.73 mg |
| Titanium Dioxide | 1.5 mg |
| Red Iron Oxide | 0.04 mg |
| Distilled Water | (15.0 mg) |

| Levocetirizine Layer | Quantity |
|---|---|
| Levocetirizine Dihydrochloride | 5.0 mg |
| Ludipress | 60.5 mg |
| Microcrystalline Cellulose | 30.0 mg |
| Croscarmellose Sodium | 3.0 mg |
| Light anhydrous silicic acid | 0.5 mg |
| Magnesium Stearate | 1.0 mg |
| Opadry White (Y-1-7000) | 3.0 mg |
| Distilled Water | (15.0 mg) |

The ingredients described in the Montelukast layer were mixed, and the mixture was pressed to a tablet using a round punch having a diameter of 5.5 mm to obtain a Montelukast tablet. Then, the Montelukast tablet was coated with a coating solution prepared by dissolving hypromellose, hydroxypropyl cellulose, titanium dioxide and red iron oxide in distilled water.

Meanwhile, the procedure of Example 1 was repeated to obtain a Levocetirizine tablet. Finally, said two tablets thus obtained were filled into a No. 1 hard capsule which is mainly composed of hypromellose, to obtain a capsule formulation comprising 10 mg of Montelukast and 5 mg of Levocetirizine.

### Example 6: Preparation of Capsule formulation VI

The procedure of Example 5 was repeated except for using a hard capsule which is mainly composed of pullulan, to obtain a capsule formulation comprising 10 mg of Montelukast and 5 mg of Levocetirizine.

### Example 7: Preparation of Capsule formulation VII

The procedure of Example 5 was repeated except for using a hard capsule which is mainly composed of gelatin, to obtain a capsule formulation comprising 10 mg of Montelukast and 5 mg of Levocetirizine.

### Example 8: Preparation of Capsule formulation VIII

| Montelukast Layer (granule) | Quantity |
|---|---|
| Montelukast Sodium | 10.4 mg (Montelukast, 10 mg) |
| D-Mannitol | 74.3 mg |
| Microcrystalline Cellulose | 74.3 mg |
| Hydroxypropyl Cellulose | 4.0 mg |
| Sodium Starch Glycolate | 30.0 mg |
| Distilled Water | (20.0 mg) |

| Levocetirizine Layer | Quantity |
|---|---|
| Levocetirizine Dihydrochloride | 5.0 mg |
| Ludipress | 60.5 mg |
| Microcrystalline Cellulose | 30.0 mg |
| Croscarmellose Sodium | 3.0 mg |
| Light anhydrous silicic acid | 0.5 mg |
| Magnesium Stearate | 1.0 mg |
| Opadry White (Y-1-7000) | 3.0 mg |
| Distilled Water | (15.0 mg) |

In accordance with the ingredients and compositions described in the Montelukast layer above, said ingredients were mixed and kneaded with a binding solution prepared by dissolving Montelukast and hydroxypropyl cellulose in distilled water, wet granulated, sieved through a 20 mesh, and dried to obtain Montelukast granules.

Meanwhile, the procedure of Example 1 was repeated to obtain a Levocetirizine tablet. Finally, the Montelukast granules and the Levocetirizine tablet were filled into a No. 1 hard capsule which is mainly composed of hypromellose, to obtain a capsule formulation comprising 10 mg of Montelukast and 5 mg of Levocetirizine.

### Example 9: Preparation of Capsule formulation IX

| Montelukast Layer | Quantity |
|---|---|
| Montelukast Sodium | 10.4 mg (Montelukast, 10 mg) |
| D-Mannitol | 74.3 mg |
| Microcrystalline Cellulose | 74.3 mg |
| Light anhydrous silicic acid | 5.0 mg |
| Hydroxypropyl Cellulose | 4.0 mg |
| Sodium Starch Glycolate | 30.0 mg |
| Magnesium Stearate | 2.0 mg |
| Hypromellose | 1.73 mg |
| Hydroxypropyl Cellulose | 1.73 mg |
| Titanium Dioxide | 1.5 mg |
| Red Iron Oxide | 0.04 mg |
| Distilled Water | (50.0 mg) |

| Levocetirizine Layer | Quantity |
|---|---|
| Levocetirizine Dihydrochloride | 5.0 mg |
| Ludipress | 60.5 mg |
| Microcrystalline Cellulose | 30.0 mg |
| Croscarmellose Sodium | 3.0 mg |
| Hydroxypropyl Cellulose | 4.0 mg |
| Distilled Water | (15.0 mg) |

The ingredients described in Montelukast layer were mixed, and the mixture was pressed to a tablet using a round punch having a diameter of 5.5 mm to obtain a Montelukast tablet. Then, the Montelukast tablet was coated with a coating solution prepared by dissolving hypromellose, hydroxypropyl cellulose, titanium dioxide, and red iron oxide in distilled water.

Meanwhile, in accordance with the ingredients and compositions described in the Levocetirizine layer above, said ingredients were mixed and kneaded with a binding solution prepared by dissolving levocetirizine and hydroxypropyl cellulose in distilled water, wet granulated, sieved through a 20 mesh, and dried to obtain Levocetirizine granules. Finally, the Montelukast tablet and the Levocetirizine granules were filled into a No. 1 hard capsule which is mainly composed of hypromellose, to obtain a capsule formulation comprising 10 mg of Montelukast and 5 mg of Levocetirizine.

### Comparative Example 1: Preparation of Complex tablet

| | Quantity |
|---|---|
| Montelukast Sodium | 10.4 mg (Montelukast, 10 mg) |
| Levocetirizine Dihydrochloride | 5.0 mg |
| D-Mannitol | 74.3 mg |
| Microcrystalline Cellulose | 74.3 mg |
| Light anhydrous silicic acid | 5.0 mg |
| Hydroxypropyl Cellulose | 4.0 mg |
| Ethanol | (20.0 mg) |
| Sodium Starch Glycolate | 30.0 mg |
| Magnesium Stearate | 2.0 mg |
| | |
| Hypromellose | 1.73 mg |
| Hydroxypropyl Cellulose | 1.73 mg |
| Titanium Dioxide | 1.5 mg |
| Distilled Water | (50.0 mg) |

In accordance with the ingredients and compositions described in the above, Montelukast sodium and Levocetirizine dihydrochloride were mixed, and the mixture was kneaded with a binding solution prepared by dissolving hydroxypropyl cellulose in ethanol, wet granulated, sieved through a 20 mesh, and dried. Subsequently, light anhydrous silicic acid and magnesium stearate were added thereto, mixed, and pressed to a tablet using a tablet machine. The resulting tablet of Montelukast and Levocetirizine was then coated with a coating solution prepared by dissolving hypromellose, hydroxypropyl cellulose, titanium dioxide and red iron oxide in distilled water, to obtain a complex tablet comprising 10 mg of Montelukast and 5 mg of Levocetirizine.

### Comparative Example 2: Preparation of Capsule formulation

The complex tablet prepared in Comparative Example 1 was filled into a hard capsule which is mainly composed of gelatin, to obtain a capsule formulation comprising 10 mg of Montelukast and 5 mg of Levocetirizine.

### Comparative Example 3: Preparation of Bilayer tablet

| | Quantity |
|---|---|
| Montelukast Sodium | 10.4 mg (Montelukast, 10 mg) |
| D-Mannitol | 74.3 mg |
| Microcrystalline Cellulose | 74.3 mg |
| Light anhydrous silicic acid | 5.0 mg |
| Hydroxypropyl Cellulose | 4.0 mg |
| Ethanol | (20.0 mg) |
| Sodium Starch Glycolate | 30.0 mg |
| Magnesium Stearate | 2.0 mg |
| | |
| Levocetirizine Dihydrochloride | 5.0 mg |
| Ludipress | 60.5 mg |
| Microcrystalline Cellulose | 30.0 mg |
| Croscarmellose Sodium | 3.0 mg |
| Light anhydrous silicic acid | 0.5 mg |
| Magnesium Stearate | 1.0 mg |
| | |
| Hypromellose | 1.73 mg |
| Hydroxypropyl Cellulose | 1.73 mg |
| Titanium Dioxide | 1.5 mg |
| Distilled Water | (50.0 mg) |

In accordance with the ingredients and compositions described in the above, said ingredients were mixed and the mixture was then kneaded with a binding solution prepared by dissolving Montelukast and hydroxypropyl cellulose in distilled water, wet granulated, sieved through a 20 mesh, and dried. Subsequently, Light anhydrous silicic acid and magnesium stearate were added thereto, mixed, and pressed to a tablet using a tablet machine.

Separately, Levocetirizine, ludipress, microcrystalline cellulose, croscarmellose sodium, light anhydrous silicic acid and magnesium stearate were mixed, and the mixture was pressed to a tablet together with the prepared Montelukast tablet to form a bilayer tablet. The bilayer tablet was then coated with a coating solution prepared by dissolving hypromellose, hydroxypropyl cellulose, titanium dioxide, and red iron oxide in distilled water, to obtain the resulting bilayer tablet comprising 10 mg of Montelukast and 5 mg of Levocetirizine.

### Experimental Example 1: Stability Test under Accelerated Conditions

The capsule formulations comprising Montelukast and Levocetirizine prepared in Examples 1, 5, 6 and 7, and Comparative Examples 1 and 2 were stored under accelerated storage conditions according to the following conditions. The content changes of Montelukast and Levocetirizine, and the amounts of related substances (impurities) were measured. The results are shown in Tables 3 to 5.

### <Accelerated Storage Conditions>

Storage conditions: Contained in an HDPE bottle at 40°C, 75% RH
Test duration: Initial, 1, 2, 4, and 6 months
Analysis target: Montelukast and Levocetirizine

### <Analysis Conditions of Montelukast and its Related substances>

Column: Zorbax SB-Phenyl column for HPLC (Agilent Zorbax) having a stainless pipe (inner diameter of 4.6 mm x length of 25 cm) filled with diisopropyl phenethyl silica gel (particle size: 5 µm)
Eluents:
A - Water containing 0.1% Trifluoroacetic acid (TFA)
B - Acetonitrile containing 0.1% TFA

**[Table 1]**

| Elution Conditions | | |
|---|---|---|
| Time (min) | A (%) | B (%) |
| 0 | 60 | 40 |
| 20 | 10 | 90 |
| 30 | 10 | 90 |
| 31 | 60 | 40 |
| 35 | 60 | 40 |

Detector: UV-absorption detector (absorbance at 238 nm)
Flow rate: 1.5 mL/min
Column temperature: 25°C

### <Analysis Conditions of Levocetirizine and its Related substances>

Column: Symmetry Shield RP18 column for HPLC (Waters) having a stainless pipe (inner diameter of 4.6 mm x length of 25 cm) filled with octadecylsilyl silica gel (particle size: 5 µm)
Eluents:
A - DW : Acetonitrile : 10% TFA = 69 : 30 : 1 (v/v)
B - DW : Acetonitrile : 10% TFA = 29 : 70 : 1 (v/v)

**[Table 2]**

| Elution Conditions | | |
|---|---|---|
| Time (min) | A (%) | B (%) |
| 0 | 100 | 0 |
| 2 | 100 | 0 |
| 30 | 25 | 75 |
| 40 | 100 | 0 |
| 50 | 100 | 0 |

Detector: UV-absorption detector (absorbance at 230 nm)
Flow rate: 1.2 mL/min
Column temperature: 30°C

The content changes of Montelukast and Levocetirizine are shown in Table 3. Also, the changes of Montelukast related substances, i.e., Montelukast sulfoxide and Montelukast *cis*-isomer, as well as the changes of Levocetirizine related substances A, B, and D are shown in Tables 4 and 5, respectively.

**[Table 3]**

| Content Changes of Montelukast and Levocetirizine | | | | | | |
|---|---|---|---|---|---|---|
| Component | Sample | Initial | 1 month | 2 month | 4 month | 6 month |
| Montelukast | Ex. 1 | 100.0 % | 99.9 % | 99.7 % | 99.7 % | 99.6 % |
| | Ex. 5 | 100.0 % | 100.0 % | 99.9 % | 99.6 % | 99.4 % |
| | Ex. 6 | 100.0 % | 99.8 % | 99.5 % | 99.4 % | 99.2 % |
| | Ex. 7 | 100.0 % | 99.8 % | 99.7 % | 99.5 % | 99.3 % |
| | Com. Ex. 1 | 100.0 % | 99.0 % | 97.6 % | 96.4 % | 93.6 % |
| | Com. Ex. 2 | 100.0 % | 98.5 % | 96.3 % | 95.4 % | 92.3 % |
| Levocetirizine | Ex. 1 | 100.0 % | 99.5 % | 99.4 % | 99.3 % | 99.2 % |
| | Ex. 5 | 100.0 % | 99.8 % | 99.6 % | 99.4 % | 99.3 % |
| | Ex. 6 | 100.0 % | 99.7 % | 99.6 % | 99.3 % | 98.9 % |
| | Ex. 7 | 100.0 % | 99.6 % | 99.5 % | 99.4 % | 99.2 % |
| | Com. Ex. 1 | 100.0 % | 98.4 % | 95.9 % | 94.4 % | 91.5 % |
| | Com. Ex. 2 | 100.0 % | 98.1 % | 94.4 % | 93.6 % | 92.7 % |

As shown in Table 3, the capsule formulations of Examples 1, 5, 6, and 7 resulted insignificant content decreases under the accelerated test condition after 6 months, and thus exhibited exceptionally good storage stability. On the contrary, the complex tablet of Comparative Example 1 prepared by simply mixing of Montelukast and Levocetirizine, and the capsule formulation of Comparative Example 2 prepared by charging the complex tablet of Comparative Example 1 into a hard capsule, showed approximately 5% or more reduction in contents over 6 months under the accelerated storage condition.

**[Table 4]**

| Changes of Montelukast Related Substances | | | | | | |
|---|---|---|---|---|---|---|
| Sample | Initial | | | Accelated condition for 6 month | | |
| | Montelukast sulfoxide (%) | Montelukast *cis*-isomer (%) | Total (%) | Montelukast sulfoxide (%) | Montelukast *cis*-isomer (%) | Total (%) |
| Ex. 1 | 0.01 | 0.02 | 0.03 | 0.09 | 0.03 | 0.15 |
| Ex.5 | 0.02 | 0.01 | 0.05 | 0.07 | 0.05 | 0.14 |
| Ex. 6 | 0.01 | 0.03 | 0.04 | 0.08 | 0.09 | 0.21 |
| Ex. 7 | 0.02 | 0.04 | 0.03 | 0.35 | 0.12 | 0.65 |
| Com. Ex. 1 | 0.04 | 0.11 | 0.20 | 2.45 | 1.35 | 4.64 |
| Com. Ex. 2 | 0.10 | 0.07 | 0.24 | 3.11 | 1.05 | 5.47 |

**[Table 5]**

| Changes of Levocetirizine Related Substances | | | | | | | | |
|---|---|---|---|---|---|---|---|---|
| Sample | Initial | | | | Accelated condition for 6 month | | | |
| | Rel. Sub. A (%) | Rel. Sub. B (%) | Rel. Sub. D(%) | Total (%) | Rel. Sub. A (%) | Rel. Sub. B (%) | Rel. Sub. D (%) | Total (%) |
| Ex. 1 | 0.02 | 0.01 | 0.01 | 0.06 | 0.12 | 0.11 | 0.02 | 0.35 |
| Ex. 5 | 0.02 | 0.01 | 0.00 | 0.05 | 0.09 | 0.11 | 0.02 | 0.42 |
| Ex. 6 | 0.01 | 0.02 | 0.01 | 0.06 | 0.08 | 0.05 | 0.03 | 0.29 |
| Ex. 7 | 0.03 | 0.06 | 0.02 | 0.12 | 0.35 | 0.38 | 0.02 | 0.85 |
| Com. Ex. 1 | 0.05 | 0.07 | 0.20 | 0.35 | 1.12 | 1.08 | 3.45 | 5.95 |
| Com. Ex. 2 | 0.04 | 0.05 | 0.15 | 0.33 | 1.11 | 1.04 | 3.05 | 6.42 |

As shown in Tables 4 and 5, the capsule formulations of Examples 1, 5, 6, and 7 resulted insignificant increases of the related substances under the accelerated test condition after 6 months, and thus exhibited exceptionally good storage stability. On the contrary, the complex tablet of Comparative Example 1 prepared by simply mixing Montelukast and Levocetirizine, and the capsule formulation of Comparative Example 2 prepared by charging the complex tablet of Comparative Example 1 into a hard capsule, showed an increase of related substances by approximately 10-fold or more under the accelerated test condition after 6 months. Therefore, it was found that a complex formulation prepared by simply mixing Montelukast and Levocetirizin degenerates its storage stability owing to the physicochemical characteristics of the active ingredients.

## Claims

1. A capsule formulation for preventing or treating allergic rhinitis and asthma, which comprises two separate layers of:
(1) a Montelukast layer comprising montelukast or a pharmaceutically acceptable salt thereof; and
(2) a Levocetirizine layer comprising levocetirizine or a pharmaceutically acceptable salt thereof,
wherein said Montelukast layer and said Levocetirizine layer contain water in an amount of 5% or less.

2. The capsule formulation of claim 1, wherein said Montelukast layer or said Levocetirizine layer is in the form of granules or a tablet.

3. The capsule formulation of claim 2, wherein at least one of said Montelukast layer and said Levocetirizine layer is in the form of a tablet.

4. The capsule formulation of claim 1, wherein said Montelukast layer and said Levocetirizine layer further comprise a pharmaceutically acceptable additive selected from the group consisting of a diluent, a disintegrant, a binder, a stabilizing agent, a lubricant, a colorant, and a mixture thereof.

5. The capsule formulation of claim 3, wherein the tablet further comprises a coating layer.

6. The capsule formulation of claim 5, wherein said coating layer comprises a coating substrate selected from the group consisting of methylcellulose, ethylcellulose, polyvinyl alcohol, polyvinylpyrrolidone, hydroxyethylcellulose, hypromellose, and a mixture thereof.

7. The capsule formulation of claim 6, wherein said coating substrate is in an amount ranging from 1 to 20% by weight based on the total weight of the tablet.

8. The capsule formulation of claim 1, wherein said capsule is a hard capsule.

9. The capsule formulation of claim 8, wherein said capsule is made from a material selected from the group consisting of hypromellose, pullulan, gelatin and polyvinyl alcohol.

10. The capsule formulation of claim 8, wherein said capsule is made from hypromellose or pullulan.

11. The capsule formulation of claim 1, wherein said montelukast or said pharmaceutically acceptable salt thereof is contained in an amount of 2.5 mg to 20 mg per unit dosage form.

12. The capsule formulation of claim 1, wherein said levocetirizine or said pharmaceutically acceptable salt thereof is contained in an amount of 2.5 mg to 20 mg per unit dosage form.

13. The capsule formulation of claim 1, wherein said pharmaceutically acceptable salt of montelukast is montelukast sodium.

14. The capsule formulation of claim 1, wherein said pharmaceutically acceptable salt of levocetirizine is levocetirizine dihydrochloride.

15. The capsule formulation of claim 1, wherein said allergic rhinitis comprises rhinorrhea, nasal obstruction, nasal itching, sneezing or ocular pruritis.

16. A method for preparing the capsule formulation of claim 1, which comprises the steps of:
(i) mixing montelukast or a pharmaceutically acceptable salt thereof and a pharmaceutically acceptable additive, and granulating the mixture to obtain granules or forming the granules into a tablet;
(ii) mixing levocetirizine or a pharmaceutically acceptable salt thereof and a pharmaceutically acceptable additive, and granulating the mixture to obtain granules or forming the granules into a tablet; and
(iii) filling said tablet or granules of montelukast prepared in step (i) and said tablet or granules of levocetirizine prepared in step (ii) into a hard capsule to form separate layers in the capsule, wherein said Montelukast layer and said Levocetirizine layer contain water in an amount of 5% or less.

17. The method of claim 16, which further comprises coating said tablet prepared in step (i) or (ii).

18. The method of claim 16, wherein at least one of said Montelukast layer and said Levocetirizine layer is in the form of a tablet.

19. The method of claim 16, wherein said Montelukast layer and said Levocetirizine layer are prepared without employing water or an organic solvent, or prepared in a condition which contains substantially no water or organic solvent

20. The method of claim 17, wherein said coating layer is prepared without employing water or an organic solvent, or prepared as a water-based coating which contains substantially no water or organic solvent.

## Patentansprüche

1. Kapselformulierung zur Verhütung oder Behandlung von allergischer Rhinitis und Asthma, die zwei separate Schichten umfasst:
(1) eine Montelukast-Schicht, die Montelukast oder ein pharmazeutisch verträgliches Salz davon umfasst; und
(2) eine Levocetirizin-Schicht, die Levocetirizin oder ein pharmazeutisch verträgliches Salz davon umfasst,
wobei die Montelukast-Schicht und die Levocetirizin-Schicht Wasser in einer Menge von 5% oder weniger enthalten.

2. Kapselformulierung nach Anspruch 1, wobei die Montelukast-Schicht oder die Levocetirizin-Schicht in Form von Granulat oder einer Tablette vorliegt.

3. Kapselformulierung nach Anspruch 2, wobei zumindest eine von der Montelukast-Schicht und der Levocetirizin-Schicht in Form einer Tablette vorliegt.

4. Kapselformulierung nach Anspruch 1, wobei die Montelukast-Schicht und die Levocetirizin-Schicht weiterhin einen pharmazeutisch verträglichen Zusatzstoff umfassen, der aus der Gruppe ausgewählt ist, die aus einem Verdünnungsmittel, einem Zerfallsmittel, einem Bindemittel, einem Stabilisator, einem Gleitmittel, einem Farbmittel und einem Gemisch davon besteht.

5. Kapselformulierung nach Anspruch 3, wobei die Tablette weiterhin eine Überzugsschicht umfasst.

6. Kapselformulierung nach Anspruch 5, wobei die Überzugsschicht ein Überzugssubstrat umfasst, das aus der Gruppe ausgewählt ist, die aus Methylcellulose, Ethylcellulose, Polyvinylalkohol, Polyvinylpyrrolidon, Hydroxyethylcellulose, Hypromellose und einem Gemisch davon besteht.

7. Kapselformulierung nach Anspruch 6, wobei das Überzugssubstrat in einer Menge im Bereich von 1 bis 20 Gew.-% bezogen auf das Gesamtgewicht der Tablette vorliegt.

8. Kapselformulierung nach Anspruch 1, wobei die Kapsel eine Hartkapsel ist.

9. Kapselformulierung nach Anspruch 8, wobei die Kapsel aus einem Material hergestellt ist, das aus der aus Hypromellose, Pullulan, Gelatine und Polyvinylalkohol bestehenden Gruppe ausgewählt ist.

10. Kapselformulierung nach Anspruch 8, wobei die Kapsel aus Hypromellose oder Pullulan hergestellt ist.

11. Kapselformulierung nach Anspruch 1, wobei das Montelukast oder das pharmazeutisch verträgliche Salz davon in einer Menge von 2,5 mg bis 20 mg pro Darreichungsformeinheit enthalten ist.

12. Kapselformulierung nach Anspruch 1, wobei das Levocetirizin oder das pharmazeutisch verträgliche Salz davon in einer Menge von 2,5 mg bis 20 mg pro Darreichungsformeinheit enthalten ist.

13. Kapselformulierung nach Anspruch 1, wobei das pharmazeutisch verträgliche Salz von Montelukast Montelukast-Natrium ist.

14. Kapselformulierung nach Anspruch 1, wobei das pharmazeutisch verträgliche Salz von Levocetirizin Levocetirizin-Dihydrochlorid ist.

15. Kapselformulierung nach Anspruch 1, wobei die allergische Rhinitis Rhinorrhoe, Nasenverstopfung, Nasenjucken, Niesen oder okulären Pruritus umfasst.

16. Verfahren zur Herstellung der Kapselformulierung nach Anspruch 1, das die Schritte umfasst:
(i) Mischen von Montelukast oder einem pharmazeutisch verträglichen Salz davon und einem pharmazeutisch verträglichen Zusatzstoff, und Granulieren des Gemischs, um Granulat zu erhalten, oder Formen des Granulats zu einer Tablette;
(ii) Mischen von Levocetirizin oder einem pharmazeutisch verträglichen Salz davon und einem pharmazeutisch verträglichen Zusatzstoff, und Granulieren des Gemischs, um Granulat zu erhalten, oder Formen des Granulats zu einer Tablette; und
(iii) Füllen der Tablette oder des Granulats von Montelukast, hergestellt in Schritt (i), und der Tablette oder des Granulats von Levocetirizin, hergestellt in Schritt (ii), in eine Hartkapsel, um separate Schichten in der Kapsel zu bilden, wobei die Montelukast-Schicht und die Levocetirizin-Schicht Wasser in einer Menge von 5% oder weniger enthalten.

17. Verfahren nach Anspruch 16, das weiterhin das Überziehen der in Schritt (i) oder (ii) hergestellten Tablette umfasst.

18. Verfahren nach Anspruch 16, wobei zumindest eine von der Montelukast-Schicht und der Levocetirizin-Schicht in Form einer Tablette vorliegt.

19. Verfahren nach Anspruch 16, wobei die Montelukast-Schicht und die Levocetirizin-Schicht ohne das Verwenden von Wasser oder einem organischen Lösungsmittel hergestellt werden, oder in einem Zustand hergestellt werden, der im Wesentlichen kein Wasser oder organisches Lösungsmittel enthält.

20. Verfahren nach Anspruch 17, wobei die Überzugsschicht ohne das Verwenden von Wasser oder einem organischen Lösungsmittel hergestellt wird, oder als Überzug auf Wasserbasis hergestellt wird, der im Wesentlichen kein Wasser oder organisches Lösungsmittel enthält.

## Revendications

1. Formulation de capsule pour prévenir ou traiter une rhinite allergique et un asthme, qui comprend deux couches séparées de:
(1) une couche de Montélukast comprenant du montélukast ou un sel pharmaceutiquement acceptable de celui-ci; et
(2) une couche de Lévocétirizine comprenant de la lévocétirizine ou un sel pharmaceutiquement acceptable de celle-ci,
dans laquelle ladite couche de Montélukast et ladite couche de Lévocétirizine contiennent de l'eau en une quantité de 5% ou moins.

2. Formulation de capsule selon la revendication 1, dans laquelle ladite couche de Montélukast ou ladite couche de Lévocétirizine est sous la forme de granules ou d'un comprimé.

3. Formulation de capsule selon la revendication 2, dans laquelle au moins l'une parmi ladite couche de Montélukast et ladite couche de Lévocétirizine est sous la forme d'un comprimé.

4. Formulation de capsule selon la revendication 1, dans laquelle ladite couche de Montélukast et ladite couche de Lévocétirizine comprennent en outre un additif pharmaceutiquement acceptable choisi dans le groupe constitué par un diluant, un désintégrant, un liant, un agent stabilisant, un lubrifiant, un colorant et un mélange de ceux-ci.

5. Formulation de capsule selon la revendication 3, dans laquelle le comprimé comprend en outre une couche d'enrobage.

6. Formulation de capsule selon la revendication 5, dans laquelle ladite couche d'enrobage comprend un substrat d'enrobage choisi dans le groupe constitué par la méthylcellulose, l'éthylcellulose, un alcool polyvinylique, une polyvinylpyrrolidone, l'hydroxyéthylcellulose, l'hypromellose et un mélange de ceux-ci.

7. Formulation de capsule selon la revendication 6, dans laquelle ledit substrat d'enrobage est en une quantité allant de 1 à 20% en poids par rapport au poids total du comprimé.

8. Formulation de capsule selon la revendication 1, dans laquelle ladite capsule est une capsule dure.

9. Formulation de capsule selon la revendication 8, dans laquelle ladite capsule est faite à partir d'une matière choisie dans le groupe constitué par l'hypromellose, le pullulane, une gélatine et un alcool polyvinylique.

10. Formulation de capsule selon la revendication 8, dans laquelle ladite capsule est faite à partir d'hypromellose ou de pullulane.

11. Formulation de capsule selon la revendication 1, dans laquelle ledit montélukast ou ledit sel pharmaceutiquement acceptable de celui-ci est contenu en une quantité de 2,5mg à 20mg par forme de dosage unitaire.

12. Formulation de capsule selon la revendication 1, dans laquelle ladite lévocétirizine ou ledit sel pharmaceutiquement acceptable de celle-ci est contenu(e) en une quantité de 2,5mg à 20mg par forme de dosage unitaire.

13. Formulation de capsule selon la revendication 1, dans laquelle ledit sel pharmaceutiquement acceptable de montélukast est le montélukast sodique.

14. Formulation de capsule selon la revendication 1, dans laquelle ledit sel pharmaceutiquement acceptable de lévocétirizine est le dichlorhydrate de lévocétirizine.

15. Formulation de capsule selon la revendication 1, dans laquelle ladite rhinite allergique comprend une rhinorrhée, une obstruction nasale, une démangeaison nasale, un éternuement ou un prurit oculaire.

16. Procédé pour préparer la formulation de capsule selon la revendication 1, qui comprend les étapes consistant:
(i) à mélanger du montélukast ou un sel pharmaceutiquement acceptable de celui-ci et un additif pharmaceutiquement acceptable, et à granuler le mélange pour obtenir des granules ou à façonner les granules en un comprimé;
(ii) à mélanger de la lévocétirizine ou un sel pharmaceutiquement acceptable de celle-ci et un additif pharmaceutiquement acceptable, et à granuler le mélange pour obtenir des granules ou à façonner les granules en un comprimé; et
(iii) à introduire ledit comprimé ou lesdits granules de montélukast préparés dans l'étape (i) et ledit comprimé ou lesdits granules de lévocétirizine préparés dans l'étape (ii) dans une capsule dure pour former des couches séparées dans la capsule, ladite couche de Montélukast et ladite couche de Lévocétirizine contenant de l'eau en une quantité de 5% ou moins.

17. Procédé selon la revendication 16, qui comprend en outre l'enrobage dudit comprimé préparé dans l'étape (i) ou (ii).

18. Procédé selon la revendication 16, dans lequel au moins l'une parmi ladite couche de Montélukast et ladite couche de Lévocétirizine est sous la forme d'un comprimé.

19. Procédé selon la revendication 16, dans lequel ladite couche de Montélukast et ladite couche de Lévocétirizine sont préparées sans employer d'eau ou de solvant organique, ou préparées dans une condition qui ne contient pratiquement pas d'eau ou de solvant organique.

20. Procédé selon la revendication 17, dans lequel ladite couche d'enrobage est préparée sans employer d'eau ou de solvant organique, ou préparée sous la forme d'un enrobage à base d'eau qui ne contient pratiquement pas d'eau ou de solvant organique.
